# EUROPÄISCHE PATENTSCHRIFT

(11) **EP 1 984 047 B1**
(45) Veröffentlichungstag und Bekanntmachung des Hinweises auf die Patenterteilung: **21.03.2012**
(21) Anmeldenummer: 07702416.4
(22) Anmeldetag: 02.02.2007
(51) Int. Cl.: A61M 15/00

(54) **VORRICHTUNG ZUM INHALIEREN EINES IN EINER KAPSEL SICH BEFINDENDEN PULVERS**
DEVICE FOR INHALING A POWDER LOCATED IN A CAPSULE
DISPOSITIF D'INHALATION D'UNE POUDRE PLACEE DANS UNE GELULE

(30) Priorität: 14.02.2006 DE 102006006647
(43) Veröffentlichungstag der Anmeldung: 29.10.2008
(73) Patentinhaber: Braunform GmbH, 79353 Bahlingen (DE)
(72) Erfinder: BELLER, Klaus-Dieter, 79341 Kenzingen (DE)
(74) Vertreter: Goy, Wolfgang
(86) Internationale Anmeldenummer: PCT/DE2007/000186
(87) Internationale Veröffentlichungsnummer: WO 2007/093149

(56) Entgegenhaltungen:
- EP-A- 1 607 117
- EP-A1- 0 707 862
- EP-A1- 1 504 780
- WO-A-97/27892
- US-A- 6 123 070

## Beschreibung

Die Erfindung betrifft eine Vorrichtung zum Inhalieren eines in einer Kapsel sich befindenden Pulvers nach dem Oberbegriff des Anspruchs 1.

Der erfindungsgemäße treibgasfreie Pulverinhalator dient zur inhalativen Applikation von pulverförmigen, feststofflichen Arzneimitteln oder von anderen Mitteln. Die Pulverinhalatoren setzten dabei das Aerosol durch den Inhalationsvorgang frei, wobei die Energie für die Dispergierung durch den inspiratorischen Fluß gewonnen wird. Die pulverförmige Substanz ist dabei in einer Kapsel enthalten.

Eine bekannte Inhalationsvorrichtung sieht ein Gehäuse vor, in welchem eine Aufnahmekammer für die Kapsel .ausgebildet ist. Die in die Aufnahmekammer eingelegte Kapsel befindet sich dabei zwischen einem Eingangsluftstrom und einem Ausgangsluftstrom, welcher durch entsprechende Kanäle in dem Gehäuse ausgebildet ist. Während des Inhalierens reißt ein durchgängiger Luftstrom das Pulver mit.

Voraussetzung hierfür ist, daß zunächst die Kapsel an den beiden Enden geöffnet wird. Neben der Perforation der beiden Enden der Kapsel durch Hineinstechen eines Hohldorns gibt es auch die Möglichkeit, die beiden Enden der Kapsel mittels einer Schneideinrichtung abzuschneiden. Hierzu dienen zwei Messer. Das Abschneiden der beiden Kapselenden verhindert zuverlässig die Entstehung von Splittern, welche in den Luftstrom gelangen könnten. Bei den bekannten Inhalationsvorrichtungen arbeitet die Schneideinrichtung dergestalt, daß sich die Kapsel bereits in ihrer Aufnahmekammer befindet und daß die beiden Messer der Schneideinrichtung in die Aufnahmekammer hineinbewegt werden, so daß die beiden Kapselenden abgeschnitten werden.

Das Problem besteht dabei darin, daß sich die Kapselnden sowie Kapselsplitter in der Aufnahmekammer befinden und es dabei zu Störungen im Luftstrom kommen kann. Auch besteht die Gefahr, daß die Kapselenden vom Luftstrom mitgerissen und vom Patienten mitinhaliert werden. Aus diesem Grunde ist es bei den bekannten Inhalationsvorrichtungen ein Sieb notwendig.

EP 1 504 780 A1 offenbart die Merkmale des Preambel des Anspruchs 1.

Davon ausgehend liegt der Erfindung die **Aufgabe** zugrunde, eine Vorrichtung zum Inhalieren eines in einer Kapsel sich befindenden Pulvers mit einer verbesserten Schneideinrichtung zum Abschneiden der beiden Enden der Kapsel zu schaffen.

Die technische **Lösung** ist gekennzeichnet durch die Merkmale im Kennzeichen des Anspruchs 1.

Die Grundidee der erfindungsgemäßen Inhalationsvorrichtung besteht darin, daß die Kapsel an den feststehenden Messern vorbeibewegt wird. Da die Schneidkanten der Messer dabei zwangsläufig entgegen der Bewegungsrichtung der Kapsel ausgerichtet sind, bleiben dort auch die abgeschnittenen Kapselenden liegen. Dadurch lassen sich die Kapselenden sehr leicht entsorgen, ohne daß die Gefahr besteht, daß sie in den Inhalationsstrom gelangen. Ein weiterer Vorteil der festen Anordnung der Messer im Gehäuse besteht darin, daß es keine Verkantungen der Messer gibt, was hingegen dann der Fall sein könnte, wenn die Messer bewegt werden. Somit ist durch die feste Anordnung der Messer im Gehäuse ein jederzeit reproduzierbares Schnittbild der Kapselenden gewährleistet.

Gemäß Anspruch 1 ist ein Pulverinhalator geschaffen, bei welchem das Pulver optimal deagglomeriert und der Entleerungsgrad deutlich verbessert ist. Die Grundidee besteht darin, daß die angesaugte Luft im Eingangsluftstrom verwirbelt und die so verwirbelte Luft anschließend dem in der Kapsel befindlichen Pulver zugeführt wird. Durch diese Verwirbelung der Luft wird das Pulver im Luftstrom fein verteilt. Dabei wird der Luftstrom gezielt auf das Pulver geleitet, wobei die Verwirbelungseinrichtung als Strömungsrichter, Verstärker sowie Verdichter wirkt.

Erfindungsgemäß ist 12 die Verwirbelungseinrichtung wendelförmig ausgebildet. Durch diese Wendelform entsteht die gewünschte Verwirbelung und gezielte Zuführung der verwirbelten Luft auf das Pulver.

Erfindungsgemäß ist die Verwirbelungseinrichtung kombiniert wendel-spiral-förmig ausgebildet. Durch diese Form der Verwirbelungseinrichtung ist eine optimale Strömungsrichtung, Verstärkung sowie Verdichtung der angesaugten Luft gewährleistet. Unter einer Wendel-Spiral-Form ist zu verstehen, daß die Wendel in Strömungsrichtung gesehen schneckenartig konisch zuläuft. Dabei befinden sich die Wendel auf einer Mittelträgerachse.

Die Weiterbildung gemäß Anspruch 2 hat den Vorteil, daß während des Einführens der Kapsel in die Aufnahmekammer des Gehäuses die Kapsel nicht nur in ihre Funktionsstellung übergeführt wird, sondern daß gleichermaßen auch die Kapselenden abgeschnitten werden. Dadurch werden durch einen Arbeitsvorgang zwei Funktionen erfüllt.

Vorzugsweise sind gemäß der Weiterbildung in Anspruch 3 die beiden Messer im Bereich der Aufnahmekammer angeordnet. Dies hat den Vorteil, daß unmittelbar nach dem Abschneiden der Kapselenden sich die so präparierten Kapseln bereits in der Aufnahmekammer befinden.

Eine vorteilhafte Weiterbildung schlägt Anspruch 4 vor. Durch den Schneidvorgang wird nicht nur zuverlässig eine Splitterbildung verhindert, es wird auch dafür gesorgt, daß die abgeschnittenen Kapselenden den Funktionsablauf des Inhalierens nicht stören, da sie nicht in die Aufnahmekammer gelangen. Dies wird erfindungsgemäß dadurch erreicht, daß in Verschieberichtung gesehen sich vor der Aufnahmekammer die Messer befinden, wobei die Messerschneiden entgegengesetzt zur Verschieberichtung der Kapsel ausgerichtet sind. Beim Hineinbewegen der Kapsel in die Aufnahmekammer werden dann die überstehenden Kapselenden abgeschnitten und kommen im Bereich zwischen dem Messer und dem Gehäuse zu liegen, und zwar außerhalb der Verschiebebahn. Dadurch ist dafür gesorgt, daß die abgeschnittenen Kapselenden nicht in den Bereich der Aufnahmekapsel gelangen können.

Eine erste Ausführungsform der beweglichen Einrichtung schlägt Anspruch 5 in Form eines linear beweglichen Schlittens vor. Dies stellt eine technisch sehr einfach zu realisierende Möglichkeit dar, um einerseits die in dem Transportschieber eingelegte Kapsel in die Aufnahmekammer überzuführen und andererseits die notwendige Bewegung zum Abschneiden der Kapselenden zu schaffen. Der Schlitten ist dabei dazu ausgelegt, daß er eine einzige Kapsel aufnehmen kann.

Die Weiterbildung gemäß Anspruch 6 schlägt eine Funktionsweise des Schlittens dahingehend vor, daß zum Einlegen der Kapsel der Schlitten aus dem Gehäuse herausgezogen wird, daß anschließend die Kapsel in den Schlitten eingelegt wird und daß schließlich der Schlitten zusammen mit der Kapsel in Richtung Gehäuse bewegt wird, so daß zum einen die Kapsel in der Aufnahmekammer des Gehäuses zu liegen kommt und wobei zum anderen während dieses Bewegungsvorganges die Kapselenden abgeschnitten werden.

Vorzugsweise weist gemäß der Weiterbildung in Anspruch 7 das Gehäuse Kammern zur Bevorratung von vollen Kapseln auf. Diese Kammern werden vorzugsweise verschlossen, damit die Kapseln nicht herausfallen.

Eine zweite, alternative Ausführungsform schlägt gemäß Anspruch 8 vor, daß die bewegliche Einrichtung eine drehbare Trommel ist, in welcher wenigstens eine Kapsel angeordnet ist.

Vorzugsweise sind gemäß der Weiterbildung in Anspruch 9 um den Umfang der Trommel herum mehrere Kammern für Kapseln vorgesehen. Die Grundidee dieser Variante besteht darin, daß sich die Kapseln wie bei einem Trommelrevolver in den Kapselkammern befinden. Durch Drehen der Trommel werden die Kapseln nacheinander in den Bereich der Aufnahmekammer im Gehäuse gebracht, nachdem sie zuvor an den Kapselenden abgeschnitten worden sind. Diese Trommel hat den Vorteil, daß zum einen mehrere Kapsel bevorratet werden können und daß zum anderen durch eine einfache Drehbewegung die jeweils nächste, volle Kapsel zum Inhalieren bereitgestellt werden kann. Vorzugsweise läßt sich die Trommel nur in eine einzige Richtung drehen, damit nicht aus Versehen durch eine falsche Drehbewegung eine bereits geleerte Kapsel in die Aufnahmekammer übergeführt wird.

Die Weiterbildung hiervon gemäß Anspruch 10 hat den Vorteil, daß von einem Basis-Pulverinhalator ausgegangen wird. In der Luftzuführungsseite wird eine spezielle Verwirbelungseinrichtung eingesetzt, und zwar entsprechend individuell dem zu verabreichenden Pulver. Dies bedeutet, daß für unterschiedliche Pulversorten unterschiedliche Verwirbelungseinrichtungen zur Verfügung stehen und wahlweise in ein und dasselbe Gehäuse eingesetzt werden können.

Eine bevorzugte Weiterbildung hiervon schlägt schließlich Anspruch 11 vor. Indem die Verwirbelungseinrichtung in einem Luftzuführungskanal mit den Randbereichen ihrer Wendel dicht anliegt, ist dadurch eine gezielte Strömungsführung des Luftstroms im Eingangsluftbereich in einer kombinierten Wendel-Spiral-Fofm gewährleistet, weil die zugeführte Luft einerseits durch die Wendel und andererseits durch seitliche Kanalbegrenzung geführt wird. Dadurch kann - wie ausgeführt - der Luftstrom exakt auf das Pulver ausgerichtet, verstärkt und verdichtet werden.

Zwei Ausführungsbeispiele einer erfindungsgemäßen Vorrichtung zum Inhalieren eines in einer Kapsel sich befindenden Pulvers werden nachfolgend anhand der Zeichnungen beschrieben. In diesen zeigt:
- Fig. 1: eine perspektivische Ansicht einer ersten Ausführungsform mit eingeschobenem Schlitten;
- Fig. 2: eine Darstellung ähnlich der in Fig. 1, jedoch mit herausgezogenem Schlitten;
- Fig. 3: eine Längsschnittdarstellung durch die Vorrichtung in Fig. 1 mit eingeschobenem Schlitten;
- Fig. 4: eine Darstellung entsprechend der in Fig. 3 mit herausgezogenem Schlitten;
- Fig. 5: eine Schnittdarstellung entsprechend der in Fig. 3, wobei jedoch die Schnittebene um 90° gedreht ist;
- Fig. 6: eine perspektivische Ansicht einer zweiten Ausführungsform mit einer Trommel;
- Fig. 7: eine Längsschnittdarstellung durch die Vorrichtung in Fig. 6;
- Fig. 8: eine Querschnittsdarstellung durch die Vorrichtung in Fig. 6.

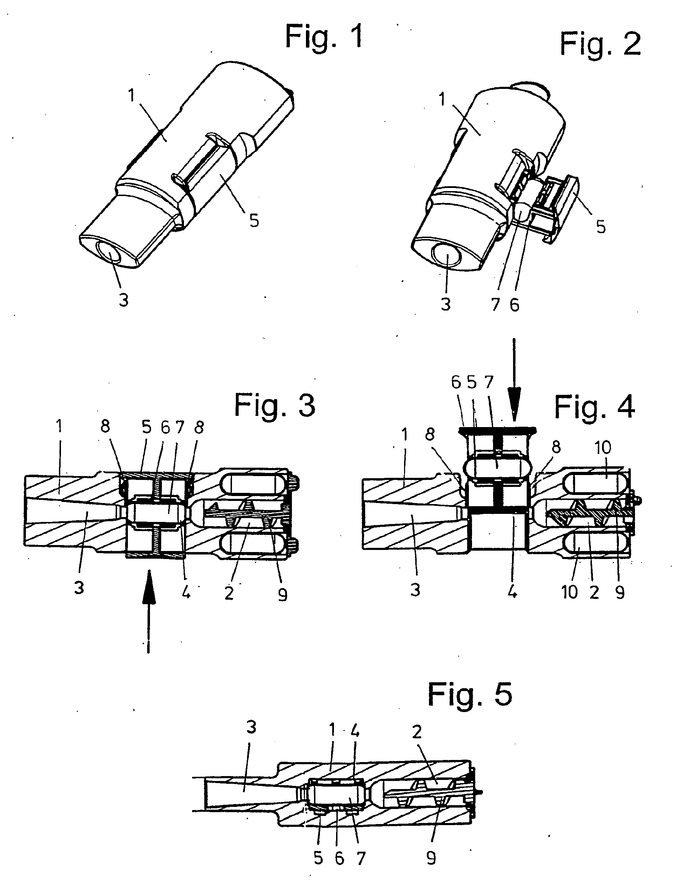

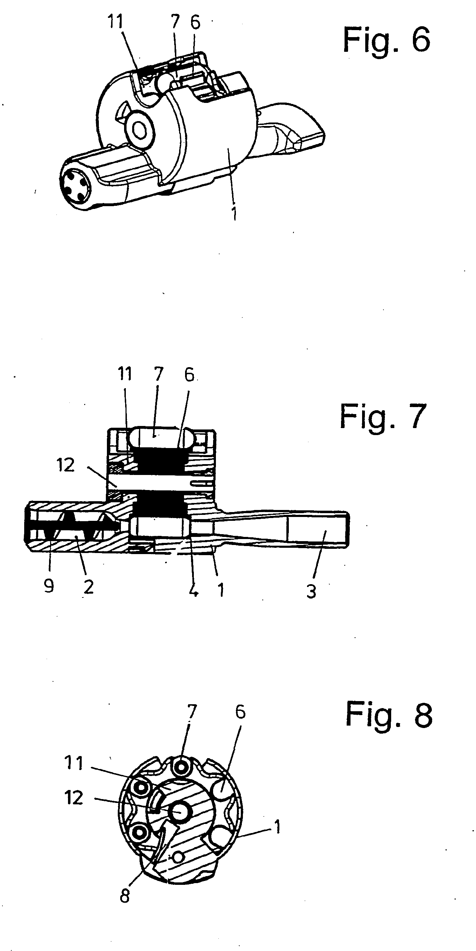

In den Fig. 1 bis 5 ist eine erste Ausführungsform und in den Fig. 6 bis 8 eine zweite Ausführungsform einer Inhalationsvorrichtung dargestellt.

Die erste Ausführungsform in den Fig. 1 bis 5 weist ein Gehäuse 1 auf. Am einen Ende dieses Gehäuses 1 befindet sich eine Lufteintrittsöffnung 2 und am anderen Ende eine Luftaustrittsöffnung 3 mit Mundstück. Dazwischen befindet sich eine Aufnahmekammer 4. Die Lufteintrittsöffnung 2, die Aufnahmekammer 4 sowie die Luftaustrittsöffnung 3 liegen dabei auf einer gemeinsamen Mittellinie.

Im Gehäuse 1 im Bereich der Aufnahmekammer 4 ist ein Schlitten 5 bezüglich des Längserstreckung des Gehäuses 1 querverschiebbar gelagert. Dieser Schlitten 5 besitzt eine Aufnahme 6 für eine Kapsel 7.

Beidseits des Schlittens 5 sind zwei Messer 8 einer Schneideinrichtung angeordnet. Der Abstand zwischen diesen beiden Messern 8 ist dabei etwas geringer als die Gesamtlänge der Kapsel 7.

Schließlich ist im Bereich der Lufteintrittsöffnung 2 eine Verwirbelungseinrichtung 9 angeordnet. Diese ist als separates Teil in die Lufteintrittsöffnung 2 eingesetzt. Die Verwirbelungseinrichtung 9 ist wendelförmig ausgebildet. Sie kann auch kombiniert wendel-spiral-förmig ausgebildet sein. Dies bedeutet, daß eine mittige Achse vorgesehen ist, welche von einer Wendel 10 umschlungen ist, wobei sich diese Wendel in Strömungsrichtung gesehen bezüglich ihres Durchmessers in der Art eines Zylinders nicht verändert. Sofern die Verwirbelungseinrichtung 9 kombiniert wendel-spiral-förmig ausgebildet ist, verringert sich hingegen die Wendel in Strömungsrichtung gesehen bezüglich ihres Durchmessers allmählich und definiert dadurch eine Art Spirale. Die Außenmantelfläche der Wendel liegt bei beiden Ausführungsformen dabei an der Innenmantelfläche der kanalförmigen Lufteintrittsöffnung 2 an.

Die Funktionsweise ist wie folgt:
Um die Inhalationsvorrichtung für die Inhalation vorzubereiten, wird der Schlitten 5 aus dem Gehäuse 1 herausgezogen (Fig. 2). Es kann dann in die Aufnahme 6 des Schlittens 5 die Kapsel 7 eingelegt werden (Fig. 2 und 4).

Anschließend wird der Schlitten 5 entsprechend der Pfeildarstellung in Fig. 4 nach innen bewegt. Dabei passieren die beiden Enden der Kapsel 7 die beiden Messer 8 dergestalt, daß die Kapselenden abgeschnitten werden. Die abgeschnittenen Kapselenden dringen dabei nicht in das Gehäuse 1 ein, sondern fallen außenseitig herunter. Im geschlossenen Zustand bei vollständig eingeschobenem Schlitten 5 befindet sich die beidseits geöffnete Kapsel 7 einerseits in der Aufnahme 6 des Schlittens 5 andererseits in der Aufnahmekammer 4 des Gehäuses 1. In dieser Position kann der Patient mit der Inhalation beginnen, indem die Luftaustrittsöffnung 3 als Mundstück zum Ansaugen dient.

Durch die speziell ausgebildete Verwirbelungseinrichtung 9 im Eingangsluftstrom wird dabei die angesaugte Luft verstärkt, verdichtet und so hinsichtlich ihrer Strömung ausgerichtet, daß die Luft exakt auf das in der Kapsel 7 befindliche Pulver geleitet wird. Dadurch wird das Pulver optimal deagglomeriert und der Entleerungsgrad optimiert. Dadurch ist ein mehrfaches Inhalieren nicht notwendig, um die Kapsel vollständig zu entleeren.

Nach der Inhalation wird der Schlitten 5 wieder herausgezogen, so daß die entleerte Kapsel 7 entnommen werden kann. Anschließend kann ein neuer Inhalationsvorgang beginnen, indem eine neue Kapsel 7 in die Aufnahme 6 des Schlittens 5 eingelegt wird. Zu diesem Zweck sind in dem Gehäuse 1 rückseitig Kammern 10 für volle Kapseln 7 vorgesehen. Diese Kammern 10 sind mittels eines Schiebers verschlossen.

Eine alternative, zweite Ausführungsform ist in den Fig. 6 bis 8 dargestellt. Auch hier ist ein Gehäuse 1 mit Lufteintrittsöffnung 2 und Luftaustrittsöffnung 3 sowie mit einer zentralen Aufnahmekammer 4 vorgesehen.

Statt eines Schlittens ist jedoch eine Trommel 11 vorgesehen, welche in dem Gehäuse 1 um eine Achse 12 drehbar gelagert ist. Diese Trommel 11 definiert um den Umfang herum eine Mehrzahl von Aufnahmen 6 für die Kapseln 7. Im Bereich der Aufnahmekammer 4 des Gehäuses 1 befinden sich wiederum zwei Messer 8.

Die Funktionsweise ist wie folgt:
Zunächst werden die Aufnahmen 6 der Trommel 11 mit vollen Kapseln 7 bestückt. Dadurch ist die Inhalationsvorrichtung betriebsbereit.

Die Trommel 11 wird in eine vorgegebene Richtung gedreht, so daß die jeweils vorderste Kapsel 7 an den Messern 8 vorbeigeführt wird, so daß die beiden Kapselenden abgeschnitten werden. Während dieses Drehvorganges gelangt die so geöffnete Kapsel 7 in die Aufnahmekammer 4 des Gehäuses 1. In dieser Position kann dann mit dem Inhalationsvorgang begonnen werden.

Sobald die Kapsel 7 entleert worden ist, kann die Trommel 11 weitergedreht und die nächste, volle Kapsel 7 in die Aufnahmekammer 4 übergeführt werden, nachdem zuvor die beiden Kapselenden mittels der Messer 8 abgeschnitten worden sind.

### Bezugszeichenliste

- 1: Gehäuse
- 2: Lufteintrittsöffnung
- 3: Luftaustrittsöffnung
- 4: Aufnahmekammer
- 5: Schlitten
- 6: Aufnahme
- 7: Kapsel
- 8: Messer
- 9: Verwirbelungseinrichtung
- 10: Kammer
- 11: Trommel
- 12: Achse

## Patentansprüche

1. Vorrichtung zum Inhalieren eines in einer Kapsel (7) sich befindenden Pulvers mit einem Gehäuse (1) mit einer Aufnahmekammer (4) für die Kapsel (7), wobei während des Inhalierens ein durchgängiger Luftstrom das Pulver mitreißt,
mit einer, im oder am Gehäuse (1) angeordneten, beweglichen Einrichtung zur Aufnahme der Kapsel (7) sowie
mit einer zwei Messer (8) aufweisenden Schneideinrichtung zum Abschneiden der beiden Enden der Kapsel (7), wobei die beiden Messer (8) der Scheideinrichtung fest im Gehäuse (1) angeordnet sind und wobei beim Bewegen der beweglichen Einrichtung vorbei an den Messern (8) die beiden Enden der Kapsel (7) abschneidbar sind,
**dadurch gekennzeichnet,**
**daß** im Eingangsluftstrom vor derjenigen Stelle, wo das Pulver in der Kapsel (7) durch den Luftstrom mitgerissen wird, eine Verwirbelungseinrichtung (9) für die angesaugte Luft vorgesehen ist,
wobei die Verwirbelungseinrichtung (9) derart kombiniert wendel-spiral-förmig ausgebildet ist, daß die Wendel in Strömungsrichtung gesehen schneckenartig konisch zuläuft.

2. Vorrichtung nach dem vorhergehenden Anspruch,
**dadurch gekennzeichnet,**
**daß** während des Abschneidvorganges die Kapsel (7) in die Aufnahmekammer (4) im Gehäuse (1) überführbar ist.

3. Vorrichtung nach Anspruch 2,
**dadurch gekennzeichnet,**
**daß** die beiden Messer (8) im Bereich der Aufnahmekammer (4) angeordnet sind.

4. Vorrichtung nach einem der vorhergehenden Ansprüche,
**dadurch gekennzeichnet,**
**daß** die beiden Messer (8) derart ausgebildet und im Gehäuse (1) angeordnet sind, daß die abgeschnittenen Enden der Kapsel (7) nicht in den Bereich der Aufnahmekammer (4) gelangen.

5. Vorrichtung nach einem der Ansprüche 1 bis 4,
**dadurch gekennzeichnet,**
**daß** die bewegliche Einrichtung ein linear beweglicher Schlitten (5) ist.

6. Vorrichtung nach Anspruch 5,
**dadurch gekennzeichnet,**
**daß** der Schlitten (5) zum Einlegen der Kapsel (7) aus dem Gehäuse (1) herausziehbar ist und
**daß** der Schlitten (5) zum Überführen der Kapsel (7) in die Aufnahmekammer (4) sowie zum Abschneiden der Enden der Kapsel (7) in das Gehäuse (1) einschiebbar ist.

7. Vorrichtung nach Anspruch 5 oder 6,
**dadurch gekennzeichnet,**
**daß** in dem Gehäuse (1) Kammern (10) zur Bevorratung von vollen Kapseln (7) vorgesehen sind.

8. Vorrichtung nach einem der Ansprüche 1 bis 4,
**dadurch gekennzeichnet,**
**daß** die bewegliche Einrichtung eine drehbare Trommel (11) ist, in welcher wenigstens eine Kapsel (7) angeordnet ist.

9. Vorrichtung nach Anspruch 8,
**dadurch gekennzeichnet,**
**daß** um den Umfang der Trommel (11) herum mehrere Aufnahmen (6) für Kapseln (7) vorgesehen sind.

10. Vorrichtung nach einem der vorhergehenden Ansprüche,
**dadurch gekennzeichnet,**
**daß** die Verwirbelungseinrichtung (9) ein in das Gehäuse (1) einsetzbares, separates Teil ist.

11. Vorrichtung nach einem der vorhergehenden Ansprüche,
**dadurch gekennzeichnet,**
**daß** die Verwirbelungseinrichtung (9) mit ihrer Umhüllenden in einem komplementären zylinderförmigen bzw. trichterförmigen Kanal (6) des Eingangsluftstroms anliegt.

## Claims

1. Device for inhaling a powder in a capsule (7),
with a housing (1) with a receiving chamber (4) for the capsule (7), wherein during inhalation a continuous air flow carries the powder,
with a movable device arranged in or on the housing (1) to receive the capsule (7), and
with a cutting device comprising two cutters (8) for cutting off the two ends of the capsule (7), wherein the two cutters (8) of the cutting device are firmly arranged in the housing (1) and wherein on movement of the movable device past the cutters (8), the two ends of the capsule (7) can be cut off,
**characterised in that** in the intake air flow from the point where the powder in the capsule (7) is carried away by the air flow, an eddy device (9) is provided for the aspirated air,
wherein the eddy device (9) is formed as a helix-spiral combination such that the helix, viewed in the flow direction, runs tapering in the manner of a worm screw.

2. Device according to the preceding claim, **characterised in that** during the cutting process the capsule (7) can be transferred into the receiving chamber (4) in the housing (1).

3. Device according to claim 2, **characterised in that** the two cutters (8) are arranged in the region of the receiving chamber (4).

4. Device according to any of the preceding claims, **characterised in that** the two cutters (8) are formed and arranged in the housing (1) such that the cut-away ends of the capsule (7) cannot reach the region of the receiving chamber (4).

5. Device according to any of claims 1 to 4, **characterised in that** the movable device is a linearly movable slide (5).

6. Device according to claim 5, **characterised in that** the slide (5) can be withdrawn from the housing (1) for insertion of the capsule (7) and that the slide (5) can be introduced into the housing (1) for transferring the capsule (7) to the receiving chamber (4) and for cutting off the ends of the capsule (7).

7. Device according to claim 5 to 6, **characterised in that** chambers (10) for storing full capsules (7) are provided in the housing (1).

8. Device according to any of claims 1 to 4, **characterised in that** the movable device is a rotatable drum (11) in which at least one capsule (7) is arranged.

9. Device according to claim 8, **characterised in that** several receivers (6) for capsules (7) are arranged around the periphery of the drum (11).

10. Device according to any of the preceding claims, **characterised in that** the eddy device (9) is a separate component which can be inserted in the housing (1).

11. Device according to any of the preceding claims, **characterised in that** the eddy device (9) with its envelope lies in a complementary cylindrical or hopper-shaped channel (6) of the inlet air flow.

## Revendications

1. Dispositif d'inhalation d'une poudre se trouvant dans une gélule (7),
avec un boîtier (1) doté d'une chambre réceptrice (4) pour la gélule (7), sachant que, pendant l'inhalation, un flux d'air continu entraîne la poudre,
avec un organe mobile disposé dans ou sur le boîtier (1) et destiné à recevoir la gélule (7),
et avec un organe de coupe présentant deux couteaux (8) et destiné à couper les deux extrémités de la gélule (7), les deux couteaux (8) de l'organe de coupe étant disposés fixement dans le boîtier (1) et les deux extrémités de la gélule (7) pouvant être coupées lorsque l'organe mobile passe le long des couteaux (8),
**caractérisé en ce qu'**un organe de tourbillonnement (9) pour l'air aspiré est prévu dans le flux d'air entrant avant l'endroit où la poudre se trouvant dans la gélule (7) est entraînée par le flux d'air,
sachant que l'organe de tourbillonnement (9) est réalisé sous une forme combinée d'hélice et de spirale de telle sorte que, considéré dans le sens d'écoulement, l'hélice se termine coniquement à la manière d'une vis sans fin.

2. Dispositif selon la revendication précédente, **caractérisé en ce que**, pendant l'opération de coupe, la gélule (7) peut être transférée dans la chambre réceptrice (4) prévue dans le boîtier (1).

3. Dispositif selon la revendication 2, **caractérisé en ce que** les deux couteaux (8) sont disposés dans la région de la chambre réceptrice (4).

4. Dispositif selon l'une des revendications précédentes, **caractérisé en ce que** les deux couteaux (8) sont configurés et disposés dans le boîtier (1) de telle sorte que les extrémités coupées de la gélule (7) ne parviennent pas dans la région de la chambre réceptrice (4).

5. Dispositif selon l'une des revendications 1 à 4, **caractérisé en ce que** l'organe mobile est un coulisseau (5) à déplacement linéaire.

6. Dispositif selon la revendication 5, **caractérisé en ce que** le coulisseau (5) peut être sorti du boîtier (1) pour mettre en place la gélule (7),
et **en ce que** le coulisseau (5) peut être rentré dans le boîtier (1) pour transférer la gélule (7) dans la chambre réceptrice (4) ainsi que pour couper les extrémités de la gélule (7).

7. Dispositif selon la revendication 5 ou 6, **caractérisé en ce que** des chambres (10) sont prévues dans le boîtier (1) pour stocker des gélules pleines (7).

8. Dispositif selon l'une des revendications 1 à 4, **caractérisé en ce que** l'organe mobile est un tambour rotatif (11) dans lequel est disposée au moins une gélule (7).

9. Dispositif selon la revendication 8, **caractérisé en ce que** plusieurs logements (6) pour des gélules (7) sont prévus tout autour de la périphérie du tambour (11).

10. Dispositif selon l'une des revendications précédentes, **caractérisé en ce que** l'organe de tourbillonnement (9) est une pièce séparée pouvant être installée dans le boîtier (1).

11. Dispositif selon l'une des revendications précédentes, **caractérisé en ce que** l'organe de tourbillonnement (9) s'applique par son enveloppante dans un canal complémentaire (6) en forme de cylindre ou d'entonnoir du flux d'air d'entrée.
